Europäisches Patentamt

European Patent Office

Office européen des brevets

(11) Publication number: **0 268 371**
**A1**

(12) **EUROPEAN PATENT APPLICATION**

(21) Application number: 87309105.2

(51) Int. Cl.⁴: **A61B 5/00**

(22) Date of filing: 15.10.87

(30) Priority: 24.10.86 GB 8625525

(43) Date of publication of application:
25.05.88 Bulletin 88/21

(84) Designated Contracting States:
AT BE CH DE ES FR GR IT LI LU NL SE

(71) Applicant: **The BOC Group plc**
**Chertsey Road**
**Windlesham Surrey GU20 6HJ(GB)**

(72) Inventor: **Halsall, David Neil**
**17 Malsis Road**
**Keighley West Yorkshire BD21 1EY(GB)**
Inventor: **Hickey, Barry Patrick Martin**
**425 Skipton Road**
**Utley Keighley West Yorkshire(GB)**

(74) Representative: **Gough, Peter**
**c/o THE BOC GROUP PLC Patent Department**
**Chertsey Road**
**Windlesham Surrey GU20 6HJ(GB)**

(54) **Detection device.**

(57) A device for sensing a swelling beneath the skin of a patient includes an optical fibre 1 a portion at least of which is releasably attached to the skin of a patient. A light source 5 sends a light signal along the optical fibre 1 and said light signal is detected by a light sensor 6. Any difference in the signal sensed by the light sensor which results from any displacement of the portion of the optical fibre attached to the skin signifies the presence of a swelling beneath the skin of the patient.

FIG. 3.

EP 0 268 371 A1

## DETECTION DEVICE

The present invention relates to devices for and methods of detecting a swelling beneath the skin of a patient.

A common problem when infusing drugs and the like into the blood vessel of a patient is that the needle or catheter tube can become dislodged from the blood vessel thereby causing infusion into the region of the blood vessel beneath the skin. This can be damaging to the surrounding tissues and in some instances dangerous to the patient.

It is an aim of the present invention to provide a device which is readily attachable to the skin of a patient and which will detect, by displacement of at least a portion of an optical fibre, any swelling beneath the skin.

According to one aspect of the present invention, a device for sensing the swelling beneath the skin of a patient comprises an optical fibre a portion at least of which is releasably attachable to the skin, a light source adjacent one end of the optical fibre and a light sensor adjacent the other end of the optical fibre, the arrangement being such that any swelling displaces said portion of the optical fibre thereby modifying the light signal emitted by the source and received by the sensor.

According to a further aspect of the present invention, a method of detecting a swelling beneath the skin of a patient comprises the steps of releasably attaching at least a portion of an optical fibre to the skin of patient, passing a light signal along the optical fibre from a light source adjacent one end of the fibre, and detecting any difference in the light signal received by a light sensor positioned adjacent the other end of the optical fibre resulting from any displacement of said portion as a result of a swelling beneath the skin.

An embodiment of the invention will now be described by way of example, reference being made to the Figures of the accompanying diagrammatic drawings in which :-

Figure 1 is a side elevation of part of a device for sensing a swelling beneath the skin of a patient;

Figure 2 is a transverse cross-section on a line II-II of Figure 1; and

Figure 3 is a schematic diagram of a device for sensing a swelling beneath the skin of a patient.

As shown, a device for sensing a swelling beneath the skin of a patient includes an optical fibre 1 at least a portion of which is arranged in the form of a loop and embedded in a support medium 2 for example, a sponge. The support medium 2 is encased in a plastics cover 3 and a thin membrane 4.

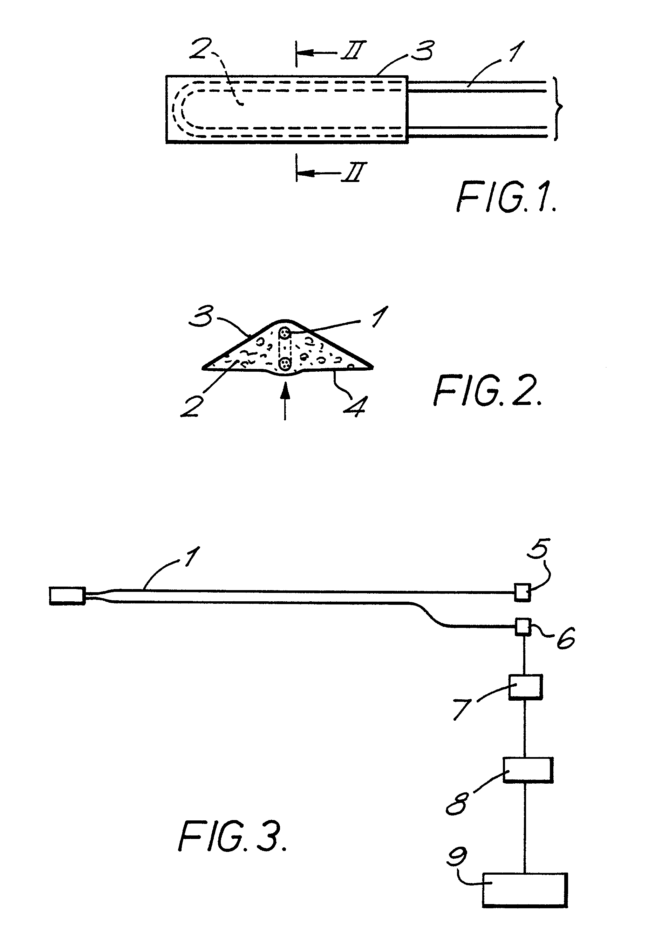

Referring in particular to Figure 3, adjacent one end of the optical fibre 1 is a light source in the form of a light emitting diode 5 and adjacent the other end of the optical fibre 1 is a light sensor 6. Electrically coupled to the light sensor 6 is an amplifier 7, a rectifier 8 and an electronic alarm 9.

In use, the portion of the optical fibre 1 embedded in the support medium 2 is releasably attached to the skin of a patient so that the thin membrane 4 is in contact with the skin of the patient. A light signal from the light emitting diode 5 is then passed along the optical fibre 1. The light signal is received by the light sensor 6 where it is converted to a small electrical AC current and then passed to amplifier 7 for amplification and rectifier 8 for rectification to give a direct current output to the electrical alarm 9. While the light signal received by the sensor 6 has a predetermined intensity the electronic alarm 9 will not be activated. However, should the light signal decrease as a consequence of pressure being applied to and displacing the portion of the optical fibre 1, as a result of any swelling under the skin beneath the said portion, then the electronic alarm will be activated.

It will be apparent that the above described embodiment is simple to manufacture and apply to a patient and will readily detect a swelling beneath the skin of a patient by using the light losses incurred by bending an optical fibre.

It will be apparent to those skilled in the art that more than one loop could be used.

**Claims**

1. A device for sensing a swelling beneath the skin of a patient characterised by an optical fibre (1) a portion at least of which is releasably attachable to the skin, a light source (5) adjacent one end of the optical fibre (1) and a light sensor (6) adjacent the other end of the optical fibre, the arrangement being such that any swelling displaces said portion of the optical fibre (1) thereby modifying the light signal emitted by the source (5) and received by the sensor (6).

2. A device as claimed in claim 1, characterised in that the said portion is in the form of a single loop.

3. A device as claimed in claim 1 or claim 2, characterised in that said portion is embedded in a support medium (2).

4. A method of detecting a swelling beneath the skin of a patient, characterised by the steps of :-

(a) releasably attaching at least a portion of an optical fibre (1) to the skin of a patient,

(b) passing a light signal along the optical fibre (1) from a light source (5) adjacent one end of the optical fibre; and

(c) detecting any difference in the light signal received by a light sensor (6) positioned adjacent the other end of the optical fibre resulting from any displacement of said portion as a result of a swelling beneath the skin.

FIG.1.

FIG.2.

FIG.3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 162 939 (SCLAVO SPA)<br>* abstract, page 2, lines 1-25; 63-72; figure 1 *<br>--- | 1,2,4 | A 61 B 5/00 |
| A | GB-A-2 132 483 (THE VICTORIA UNIVERSITY OF MANCHESTER)<br>* abstract, figure 1 *<br>--- | 1-4 | |
| A | US-A-4 112 923 (J.J. TOMECEK)<br>* column 5, lines 9-24; column 6, lines 43-68; figures 7, 12 *<br>--- | 1-4 | |
| A | US-A-4 468 197 (W. PROVOST)<br>* abstract, figure 1 *<br>----- | 1-4 | |

TECHNICAL FIELDS
SEARCHED (Int. Cl.4)

A 61 B 5/00

The present search report has been drawn up for all claims

| Place of search | Date of completion of the search | Examiner |
|---|---|---|
| BERLIN | 14-01-1988 | WEIHS J.A. |

EPO FORM 1503 03.82 (P0401)